(19) Europäisches Patentamt
European Patent Office
Office européen des brevets

(11) Veröffentlichungsnummer : **0 036 129**
**B1**

(12) # EUROPÄISCHE PATENTSCHRIFT

(45) Veröffentlichungstag der Patentschrift : **13.06.84**

(51) Int. Cl.³ : **C 07 D233/58**, C 07 D233/56

(21) Anmeldenummer : **81101538.7**

(22) Anmeldetag : **04.03.81**

(54) **Verfahren zur Herstellung von Imidazolen.**

(30) Priorität : **13.03.80 DE 3009631**

(43) Veröffentlichungstag der Anmeldung : **23.09.81 Patentblatt 81/38**

(45) Bekanntmachung des Hinweises auf die Patenterteilung : **13.06.84 Patentblatt 84/24**

(84) Benannte Vertragsstaaten : **BE CH DE FR GB LI**

(56) Entgegenhaltungen :
**EP-A- 0 000 208**
**FR-A- 2 165 514**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Schwarz, Helmut, Dr.**
**Weinbrennerstrasse 5**
**D-6700 Ludwigshafen (DE)**
Erfinder : **Dockner, Toni, Dr.**
**Grossgasse 6**
**D-6701 Meckenheim (DE)**
Erfinder : **Kempe, Uwe, Dr.**
**Carl-Bosch-Strasse 44**
**D-6703 Limburgerhof (DE)**
Erfinder : **Krug, Herbert**
**Mussbacher Strasse 49**
**D-6700 Ludwigshafen (DE)**
Erfinder : **Praetorius, Werner, Dr.**
**Dirmsteiner Weg 47**
**D-6700 Ludwigshafen (DE)**
Erfinder : **Magnussen, Peter, Dr.**
**Professor-Dillinger-Strasse 25**
**D-6702 Bad Duerkheim 1 (DE)**
Erfinder : **Gallei, Ewald, Dr.**
**August-Bebel-Strasse 48**
**D-6806 Viernheim (DE)**
Erfinder : **Fehr, Erich, Dr.**
**Kösterkamp 7**
**D-4442 Salzbergen (DE)**

**EP 0 036 129 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von Imidazolen durch Umsetzung von 2-Imidazolinen bei Temperaturen von 250 bis 500 °C in Gegenwart von Molybdänoxid Nickeloxid und/oder Kobaltoxid sowie Aluminiumoxid, Aluminiumsilikat, Siliciumdioxid, Magnesiumaluminium silikat hydrat, Dimagnesium aluminium silikathydrat, Natrium aluminiumsilikat, Calciumaluminium silikat, und/oder Magnesium silikat als Katalysatoren.

Es ist aus der DE-A- 19 52 991 bekannt, daß man Alkylendiamine mit Carbonylverbindungen in Gegenwart eines Kupfer- und/oder Chrom-Katalysators bei 320 bis 650 °C zu Imidazolen umsetzen kann. Wie alle Beispiele zeigen, muß zusätzlich Wasserstoff der Reaktion zugeführt werden ; wieder verwertbare Nebenprodukte werden nicht erhalten.

Die US-A- 2 847 417 beschreibt die Herstellung von Imidazolen aus Alkylendiaminen und Carbonsäuren an Trägerkatalysatoren der Platinmetalle. Solche Katalysatoren sind teuer und werden durch bestimmte Stoffe wie Schwefel, Schwermetalle oder Halogenide leicht vergiftet. Edelmetallkatalysatoren verlangen aufwendige Herstellungsverfahren, um die gleichmäßige Wirksamkeit des Katalysators zu gewährleisten, sowie sehr reine Ausgangsstoffe, um die Wirkung des Katalysators nicht zu beeinträchtigen. Wie Beschreibung (Spalte 1, Zeilen 50 bis 70, Spalte 2, Zeilen 65 bis 70) und die Beispiele zeigen, wird auch bei diesem Verfahren zusätzlich Wasserstoff zugesetzt, um die Bildung teeriger Polymerer und Ablagerungen auf dem Katalysator zu vermeiden und die Wirksamkeit des Katalysators so möglichst lange zu erhalten.

Es ist aus der SU-A- 201 418 bekannt, daß man 2-Methylimidazolin in Gegenwart eines Metallkatalysators und mit Diphenyloxid als Reaktionsmedium bei WB/BL einer Temperatur von 180 bis 230 °C zu 2-Methylimidazol dehydriert. Die Patentschrift zeigt in einem Beispiel, daß Mengen von 47 Gramm Ausgangsstoff umgesetzt werden. Führt man die Umsetzung in der flüssigen Phase im großtechnischen Maße durch, erhält man eine wesentlich geringere Ausbeute bis zu weniger als 60 % der Theorie. Ein erheblicher Anteil an Imidazol wird durch Dealkylierung gebildet, und zwar um so mehr, je höher der Umsatz zu 2-Methylimidazol liegt. Das Verfahren ist daher im Hinblick auf Einsparung teuerer Lösungsmittel, gute Ausbeute und einfachen und wirtschaftlichen Betrieb unbefriedigend. Ein hoher Umsatz wäre andererseits erstrebenswert, weil so die destillative Abtrennung des unumgesetzten Imidazolins nicht mehr erforderlich ist. Ein solcher Vorteil wäre besonders im Falle der Herstellung hochschmelzender Imidazole, z. B. des 1,2-Diphenylimidazols, von Bedeutung.

In den DE-A-27 29 017, DE-A-27 28 976, und DE-A-27 33 466 sowie der EP-A-0 000 208 sind Verfahren zur Herstellung von Imidazolen durch Umsetzung von 2-Imidazolinen bei einer Temperatur von 300 bis 600 °C in Gegenwart von Zinkoxid oder einem Gemisch von Zinkoxid und Aluminiumoxid als Katalysatoren beschrieben.

Nach den Angaben der DE-C 12 31 249 läßt sich Imidazol durch Umsetzung von N,N'-Diformyläthylendiamin in der Gasphase an Zinkoxidkatalysatoren bei Temperaturen zwischen 250 und 800 °C herstellen. Die Imidazolausbeute beträgt bei diesem Verfahren zwischen 42 % und 80 % der Theorie. Alle Beispiele zeigen, daß zusätzlich Stickstoff als Schleppmittel oder Verdünnungsmittel zugeführt wird, wobei die Konzentration von 1 bis 999 Mol Stickstoff je Mol Ausgangsstoff beträgt. Nach den Angaben der Patentschrift sind die Ausbeuten bei Verwendung von Zinkoxid als dem einzigen Katalysator unbefriedigend. Wie die Beschreibung (Spalte 2, Zeilen 29 bis 36) zeigt, werden die Katalysatoren in einfacher Weise hergestellt und weisen keine besonderen Strukturmerkmale, lediglich eine Korngröße von 0,2 bis 0,4 mm auf. Im Hinblick auf das in der US-A- 2 847 417 beschriebenen Verfahren, dessen Ausbeuten in der Beschreibung als sehr gering angegeben werden, wird kein Wasserstoff verwendet ; in allen Beispielen kommt Stickstoff in einer Menge von 45 bis 60 Mol je Mol Ausgangsstoff zur Anwendung. Die beste Ausbeute an Imidazol von 80 % (Beispiel 2) wird mit einem Katalysator von 78 Gewichtsprozent Zinkoxid, 7 Gewichtsprozent Aluminiumoxid, 5 Gewichtsprozent Calciumoxid, 5 Gewichtsprozent Kaliumsulfat, 2 Gewichtsprozent Magnesiumoxid, 1 Gewichtsprozent Chrom-III-oxid, 2-Gewichtsprozent Eisen-III-oxid, Natriumoxid und Kaliumoxid bei 500 °C in Gegenwart von Stickstoff erhalten.

Die nachgängige DE-A-27 29 017 verwendet im Gegensatz zu dem Verfahren der DE-C-12 31 249 keine N,N'-Diformyl-1,2-diamine, sondern setzt 1,2-Diamine mit Ameisensäure an Zinkoxid bzw. an Zinkoxid und Aluminiumoxid, bevorzugt von bestimmter Struktur, bei 300 bis 600 °C um. Es wird ausdrücklich festgestellt, daß bei diesem Katalysator ein Zusatz von Wasserstoff nicht zweckmäßig und auch nicht notwendig ist, da eine Bildung teeriger Polymere, Ablagerungen auf dem Katalysator und ein rascher Rückgang der Katalysatoraktivität nicht beobachtet werden. Ein Vergleich der Herstellungsweise von Imidazol (Beispiel 2) mit der besten Verfahrensweise der DE-A-12 31 249 (Beispiel 2) zeigt, daß der Zinkoxid/Aluminiumoxid-Katalysator auch in Gegenwart von Stickstoff und einer Temperatur von 550 °C eine wesentlich schlechtere (69,2 %) Ausbeute als der aus 9 Komponenten und beliebig strukturiertem Zinkoxid gebildete Katalysator im Beispiel 2 der Patentschrift liefert.

Die FR-A-1 266 702 beschreibt die Umsetzung eines Gemisches von einem Alkylendiamin und Formaldehyd bei vorteilhaft 315 bis 431 °C an einem Edelmetallkatalysator auf Träger. In allen Beispielen wird ein Platinkatalysator auf Aluminiumoxid verwendet. Die Ausbeuten sind unbefriedigend. Es wird erwähnt, daß zwar Kobaltmolybdat auf einem Träger als Katalysator verwendet werden kann, aber darauf

aufmerksam gemacht (Seite 2, letzter Absatz), daß nicht notwendigerweise gleich gute Ergebnisse erhalten werden. Die Patentschrift erläutert, daß nur die echte Verbindung Kobaltmolybdat und kein Gemisch von Kobaltoxid und Molybdänoxid verwendet werden können. Als Träger wird Aluminiumoxid angegeben.

Es wurde nun gefunden, daß man Imidazole der Formel (I)

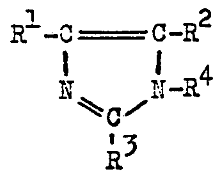

(I)

worin $R^1$, $R^2$, $R^3$ und $R^4$ gleich oder verschieden sein können und jeweils einen Alkylrest mit 1 bis 18 Kohlenstoffatomen, einen Allylrest, einen Crotylrest, einen Oleylrest, einen n-Undecen-(11)-yl-(1)-rest, einen Cycloalkylrest mit 5 bis 7 Kohlenstoffatomen, einen Aralkylrest oder Alkylarylrest mit 7 bis 12 Kohlenstoffatomen, einen Phenylrest oder ein Wasserstoffatom bedeuten, durch Umsetzung von Imidazolinen in Gegenwart von Metallkatalysatoren bei einer Temperatur von 250 bis 500 °C vorteilhaft erhält, wenn man 2-Imidazoline der Formel (II)

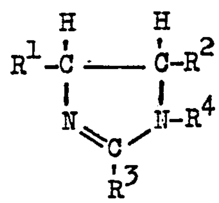

(II)

worin $R^1$, $R^2$, $R^3$ und $R^4$ die vorgenannte Bedeutung besitzen, in Gegenwart von Inertgas oder solche Inertgase enthaltendem Kreisgas und in Gegenwart eines Katalysators oder gegebenenfalls Trägerkatalysators, dessen aktive Komponente aus

a) Molybdänoxid,

b) Nickeloxid und/oder Kobaltoxid,

c) Aluminiumoxid, Aluminiumsilikat, Siliziumdioxid, Magnesiumaluminiumsilikathydrat, Dimagnesiumaluminiumsilikat, Natriumaluminiumsilikat, Calciumaluminiumsilikat und/oder Magnesiumsilikat im Molverhältnis a : b : c wie 1 : (0,5-5) : (5-50) und

d) gegebenenfalls Sauerstoffverbindungen des Zinks, Magnesiums, Chroms, Phosphors und/oder Natriumoxid besteht, umsetzt wobei die Katalysatormenge pro Mol Ausgangs-2-Imidazolin der Formel (II) 0,1-1,5 Mol Molybdänoxid entspricht.

Weiterhin wurde gefunden, daß man das Verfahren vorteilhaft ausführen, kann man in einem ersten Schritt N,N'-Diformyl-1,2-diamine der Formel (III)

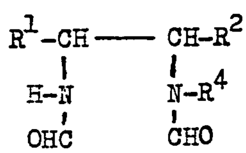

(III)

worin $R^1$, $R^2$ und $R^4$ die vorgenannte Bedeutung besitzen, bei einer Temperatur von 200 bis 400 °C in Gegenwart von Zinkoxid und/oder Aluminiumoxid als Katalysatoren in der Gasphase umsetzt und dann das so erhaltene, 2-Imidazolin (II) enthaltende Reaktionsgemisch in einem zweiten Schritt nach dem Verfahren nach Anspruch 1 umsetzt.

Weiterhin wurde gefunden, daß man das Verfahren vorteilhaft ausführt, wenn die Umsetzung in Gegenwart von Katalysatoren mit einer Teilchengröße von 0,05 bis 7 Millimeter durchgeführt wird.

Weiterhin wurde gefunden, daß man das Verfahren vorteilhaft ausführt, wenn die Umsetzung im Festbett mit Verweilzeiten der Ausgangsstoffe (II) in der Katalysatorschicht von 0,2 bis 20 Sekunden und der Wirbelschicht mit Verweilzeiten von 0,01 bis 20 Sekunden durchgeführt wird.

Die Umsetzunf kann für den Fall der Verwendung von 1,2-Diphenylimidazolin durch die folgenden Formeln wiedergegeben werden :

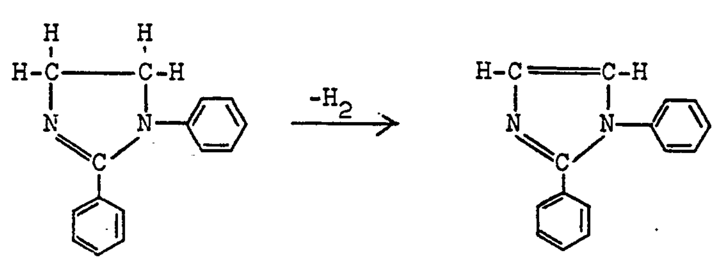

Im Vergleich zu den bekannten Verfahren liefert das Verfahren nach der Erfindung auf einfacherem und wirtschaftlicherem Wege Imidazole in guter Ausbeute und Reinheit. Schwefel- und Blausäureverbindungen als Reaktionskomponenten werden vermieden, jedoch überraschend hohe Ausbeuten erzielt. Bei Unterdruck muß nicht umgesetzt werden. Vorteilhaft ist die Arbeitsweise in der Gasphase ; so können 2-Imidazoline, die in flüssiger Phase unbeständig sind, auch ohne Isolierung weiter umgesetzt werden. Auch wenn kein zusätzlicher Wasserstoff zugeführt wird, werden eine Bildung teeriger Polymere, Ablagerungen auf dem Katalysator, Zusammenbacken des Katalysators und ein rascher Rückgang der Katalysatoraktivität nicht beobachtet. Auch nach 2 000 Stunden Betriebszeit befindet sich der Katalysator in einem rieselfähigen Zustand. Durch die langen Betriebszeiten erhöht sich die Raum-Zeit-Ausbeute. Die verwendeten Katalysatoren zeigen nach der Regenerierung keine Änderung in Bezug auf die Lebensdauer. Die Katalysatorbelastungen, zweckmäßig 100 bis 300 g Ausgangsstoff II/Liter Katalysator und Stunde, bleiben nach der Regenerierung ebenfalls praktisch unverändert. Ein Zusatz von Wasserstoff ist nicht notwendig noch zweckmäßig. Im Vergleich zu den Katalysatoren der bekannten Verfahren sind die erfindungsgemäßen Katalysatoren billiger, leichter regenerierbar und werden nicht in deutlichem Maße während einer längeren Betriebsdauer vergiftet. Gerade auch im großtechnischen Maßstab ist das Gesamtergebnis mit Bezug auf Umsatz und Ausbeute im Vergleich zum Stand der Technik besser. Alle diese vorteilhaften Eigenschaften sind im Hinblick auf den Stand der Technik überraschend.

Als Ausgangsstoffe II kommen z. B. in Frage : In 2-Stellung, 4-Stellung oder 5-Stellung einfach oder in 2 dieser Stellungen gleich oder unterschiedlich zweifach oder in diesen 3-Stellungen gleich oder unterschiedlich dreifach durch die Methyl-, Äthyl-, Allyl-, Crotyl-, Propyl-, Isopropyl-, Butyl-, Isobutyl-, Pentyl-, sek.-Butyl-, tert.-Butyl-, Hexyl-, Heptyl-, Octyl-, Oleyl-, n-Undecen-(11)-yl-(1), Nonyl-, Decyl-, Octadecyl-, Benzyl-, Cyclohexyl-, Cyclopentyl-, Toluyl-, Xylyl-, Naphthyl-, Äthylphenyl-, Phenyl-gruppe substituierte 1-Phenyl-2-imidazoline, unsubstituiertes 1-Phenyl-2-imidazolin ; homologe, in 1-Stellung durch die Methyl-, Äthyl-, Allyl-, Crotyl-, Propyl-, Isopropyl-, Butyl-, Isobutyl-, Pentyl-, sek.-Butyl-, tert.-Butyl-, Hexyl-, Heptyl-, Octyl-, Oleyl-, n-Undecen-(11)-yl-(1)-, Nonyl-, Decyl-, Octadecyl-, Cyclohexyl-, Cyclopentyl-, Benzyl-, Tolyl-, Xylyl-, Naphthyl-, Äthylphenyl-gruppe substituierte 2-Imidazoline ; unsubstituiertes Imidazolin und in 1-Stellung unsubstituierte, in 2-, 4- und/oder 5-Stellung in vorgenannter Weise substituierte Imidazoline.

Die Umsetzung wird bei einer Temperatur von 250 bis 500 °C, zweckmäßig von 280 bis 480 °C, vorzugsweise von 320 bis 410 °C, drucklos oder unter Druck, kontinuierlich oder diskontinuierlich durchgeführt. in der Regel dient das Reaktionsgemisch auch als Lösungsmedium, gegebenenfalls können auch unter den Reaktionsbedingungen inerte, organische, zweckmäßig mit Wasser kein Azeotrop bildende Lösungsmittel, z. B. aliphatische Kohlenwasserstoffe wie Petroläther oder Ligroin, verwendet werden. Unter den organischen Lösungsmitteln sind solche mit einem Siedepunkt über 120 °C, zweckmäßig über 140 °C, z. B. entsprechende Benzinfraktionen mit Siedepunlit von 120 bis 160 °C, bevorzugt.

Der Katalysator enthält stets 3 Komponenten, nämlich a) Molybdänoxid, b) Nickeloxid allein, Kobaltoxid allein oder ein Gemisch beider Oxide, c) Aluminiumoxid, Aluminiumsilikat, Siliciumdioxid, Magnesiumaluminiumsilikathydrat, Dimagnesiumaluminiumsilikathydrat, Natriumaluminiumsilikat, Calciumaluminiumsilikat oder Magnesiumsilikat allein oder ein Gemisch von 2 oder mehr dieser Verbindungen. Man verwendet Nickeloxid und/oder Kobaltoxid zu Molybdänoxid in einem Verhältnis von 0,5 bis 5, vorzugsweise 1 bis 2,6 Mol Nickeloxid und/oder Kobaltoxid je Mol Molybdänoxid, und ein Verhältnis von Aluminiumoxid, Aluminiumsilikat, Siliciumdioxid, Magnesiumaluminiumsilikathydrat, Dimagnesiumaluminiumsilikathydrat, Natriumaluminiumsilikat, Calciumaluminiumsilikat und/oder Magnesiumsilikat von 5 bis 50, vorzugsweise 8 bis 12 Mol Aluminiumoxid, Aluminiumsilikat, Siliciumdioxid, Magnesiumaluminiumsilikathydrat. Dimagnesiumaluminiumsilikathydrat, Natriumaluminiumsilikat, Calciumaluminiumsilikat und/oder Magnesiumsilikat je Mol Molybdänoxid. Es werden von 0,1 bis 1,5, vorzugsweise von 0,2 bis 0,6 Mol Molybdänoxid je Mol Ausgangsstoff (II) verwendet.

Der Katalysator kann außerdem gegebenenfalls noch Sauerstoffverbindungen des Zinks, Magnesiums, Chroms, Phosphors enthalten, vorzugsweise Zinkoxid, Magnesiumoxid, Chrom-III-oxid, Natriumchromat, Natriumbichromat, Chromtrioxid, Phosphorsäure, Phosphorpentoxid, Natrium- und Kaliumphosphat, zweckmäßig in einem Verhältnis von 0,6 bis 20, vorzugsweise 4 bis 10 Gewichtsprozent Sauerstoffverbindungen des Zinks, Magnesiums, Chroms und/oder Phosphors, bezogen auf das Gesamtgewicht des Katalysators ohne Träger oder 0,5 bis 15, vorzugsweise 3 bis 7 Gewichtsprozent Sauerstoffverbindungen vorgenannter Metalle, bezogen auf das Gesamtgewicht des Katalysators mit Träger.

Anstelle von Kobalt-, Nickel- und Molybdänoxid können auch soche Verbindungen der 3 Metalle, die unter den Reaktionsbedingungen Oxide bilden, verwendet werden. Geeignet sind z. B. die Hydroxide, Carbonate, Bicarbonate, Sulfate, Nitrate, Chloride, Bromide, Acetate oder Formiate von Nickel, Kobalt und Molybdän.

Als Aluminiumoxid kommen z. B. α- und γ-Aluminiumoxid in Frage. Man kann auch Zinkverbindungen, die unter den Reaktionsbedingungen die vorgenannte Zusatzkomponente Zinkoxid ergeben, verwenden, z. B. ein mit Zinkchlorid oder Zinksulfat imprägniertes Aluminiumoxid. Ebenfalls kommen anstelle von Aluminiumoxid auch dieses Oxid enthaltende Stoffe bzw. Stoffgemische in Betracht, z. B. Fullererde, Tone, Bleicherden wie Bentonit, Bauxit, Bimsstein, Andalusit, Kaolin, Allophane, Zeolithe,

Mullit, Korund, γ-Tonerde, Hydrargillit, Böhmit. Als Siliciumdioxid kommen zweckmäßig in Frage Kieselsäureverbindungen, Montmorillonit, Quarz, Asbest ; gefällte Kieselsäure, Kieselgel, Kieselgur.

Der Katalysator kann trägerfrei sein oder auch auf einem Träger, vorteilhaft in einer Menge von 1 bis 18 Gewichtsprozent Katalysator, bezogen auf den Träger, aufgebracht sein. Zweckmäßig werden vorgenannte Silicium- und Aluminiumverbindungen gleichzeitig in Gestalt des darin enthaltenen $SiO_2$ und/oder $Al_2O_3$ als Katalysatorkomponente und für die Oxide der Komponenten a) und b) als Träger dienen. Jedoch können auch Träger wie Titandioxid, Zirkondioxid, Zinndioxid, Aktivkohle ; Erdalkalisulfate oder Erdalkaliphosphat, z. B. die Calcium- oder Bariumsalze ; oder entsprechende Gemische vorgenannter Trägermaterialien verwendet werden. Bevorzugte Träger sind Aluminiumoxid, Magnesiumoxid und Silikate.

Die Herstellung der Trägerkatalysatoren wird nach den üblichen Verfahren, z. B. durch Auftragen der Nickel- und/oder Kobaltverbindung, Molybdänverbindung und gegebenenfalls der Silicium- und/oder Aluminiumverbindung auf den Träger, Trocknen und Calcinieren, beispielsweise zwischen 400 und 1 200 °C in reduzierender, oxidierender oder inerter Atmosphäre, durchgeführt. Der Träger, z. B. Aluminiumoxid, kann auch in seiner gewünschten geometrischen Form mit der Lösung einer der vorgenannten Verbindungen allein bzw. der Nickel- und/oder Kobalt- und Molybdänverbindung, z. B. einer wäßrigen Lösung von Nickelsulfat und Molybdänsulfat, getränkt und getrocknet werden. Ebenfalls kann man das Trägermaterial in Gestalt der Komponenten c) mit der Nickel- und/oder Kobaltverbindung und gegebenenfalls der Molybdänverbindung und Wasser verkneten, in die gewünschte Form bringen, trocknen und bei einer Temperatur von 400 bis 1 200 °C calcinieren.

Die Teilchengröße der Katalysatoren mit und ohne Träger beträgt vorzugsweise von 0,05 bis 7, insbesondere von 2 bis 4 Millimetern. Die Form kann beliebig, z. B. in Pillen-, Zylinder- oder Strangform, kugelförmig oder körnig, gewählt werden. Bevorzugt sind bei dem Katalysator auf Träger Porenvolumina von 0,05 bis 1 Milliliter je Gramm, spezifische Oberflächen von 1 bis 300 Quadratmeter je Gramm und Schüttgewichte von 0,4 bis 2,1 Gramm je Milliliter.

Vorzugsweise werden die Katalysatoren allein oder auf dem Träger in Plitt- oder Kugelform in der Wirbelschicht eingesetzt, wobei zweckmäßig Katalysatorteilchen mit Korngrößen von 0,005 bis 3 mm, insbesondere von 0,1 bis 1 mm, bevorzugt 0,2 bis 0,4 mm, verwendet werden. Die Schichtöhe des Katalysatorbettes im Wirbelzustand beträgt vorteilhaft 30 bis 2 000, insbesondere 60 bis 80 Millimeter oder wird zweckmäßig so gewählt, daß sich Verweilzeiten der Ausgangsstoffe II in der Katalysatorschicht von 0,01 bis 20, vorzugsweise von 5 bis 10 Sekunden ergeben. Bezüglich der Herstellung der Katalysatoren wird auf Houben-Weyl, Methoden der Organischen Chemie, Band 4/2, Seiten 142 ff und Ullmanns Encyklopädie der technischen Chemie, Band 9, Seiten 271 ff, verwiesen.

Die Umsetzung wird vorteilhaft in Gegenwart von Inertgasen durchgeführt. Als unter den Reaktionsbedingungen inerte Gase (Inertgas) werden zweckmäßig Edelgase wie Xenon, Argon, Neon, Helium ; Alkane wie Methan, Äthan, Propan, 2,2-Dimethylpropan, Butan, Pentan, Isobutan ; bevorzugt Stickstoff, Kohlenmonoxid und/oder Kohlendioxid ; und entsprechende Gemische, insbesondere auch $CO/H_2$-Gemische verwendet. Man verwendet zweckmäßig Mengen von 5 bis 100 Mol Inertgas, insbesondere 20 bis 80 Mol Stickstoff oder $CO/H_2$-Gemische in Mengen von 1 bis 100, vorteilhaft 2 bis 50, insbesondere 3 bis 18 Mol Kohlenmonoxid und 1 bis 100, vorteilhaft 3 bis 50, insbesondere 6 bis 20 Mol Wasserstoff je Mol Ausgangsstoff II. Unter Mol Inertgas wird im Falle von Gasgemischen das durchschnittliche Mol des Gemischs verstanden. Die Gemische können noch weitere gasförmige bzw. dampfförmige Komponenten enthalten. Bevorzugt wird das Abgas der erfindungsgemäßen Reaktion ganz oder teilweise zurückgeführt (Kreisgas) und als Gasgemisch von $CO/H_2$ verwendet. Vorteilhaft ist ein Kreisgas von 30 bis 90, insbesondere 50 bis 75 Gewichtsprozent CO, von 3 bis 15, insbesondere 5 bis 7 Gewichtsprozent Wasserstoff, das im allgemeinen noch von 0 bis 50, insbesondere 10 bis 40 Gewichtsprozent $CO_2$, 0 bis 30, insbesondere von 0 bis 10 Gewichtsprozent Stickstoff, 0 bis 10, insbesondere 0,5 bis 5 Gewichtsprozent Ammoniak, 0 bis 15, insbesondere 0,5 bis 10 Gewichtsprozent dem Ausgangsstoff II zugrundeliegendes Diamin, 0 bis 12, insbesondere 0,1 bis 6 Gewichtsprozent Methan enthält.

Die Reaktion kann wie folgt durchgeführt werden : Der feste, verflüssigte oder zweckmäßig dampfförmige Ausgangsstoff II wird im Gemisch mit Inertgas, zweckmäßig in Form von Stickstoff oder des Abgases der Reaktion als Kreisgas, bei der Reaktionstemperatur über den Katalysator bzw. Katalysator auf dem Träger in einem Festbett geleitet. Das aus dem Reaktor dampfförmig austretende Reaktionsgemisch wird gegebenenfalls in einem Zyklon entstaubt und in einer gekühlten Vorlage kondensiert. Durch fraktionierte Destillation wird dann zweckmäßig der Endstoff abgetrennt. Der Endstoff kann auch durch Umkristallisation oder Umfällung aus geeigneten Lösungsmitteln, z. B. mit Toluol, Dimethylformamid, oder verdünnten Säuren, z. B. mit Ameisensäure, isoliert werden. Die Verweilzeiten im Festbett betragen zweckmäßig von 0,2 bis 20 Sekunden.

In einer bevorzugten Ausführungsform des Verfahrens wird der Ausgangsstoff II in einer Wirbelschicht bei der Reaktionstemperatur umgesetzt. Der Katalysator bzw. Katalysator auf Träger kann durch das Ausgangsgemisch oder das Inertgas allein, zweckmäßig in Gestalt von Stickstoff oder des vorgenannten Kreisgases, als Wirbelschichtgas bei Normaldruck oder vermindertem oder erhöhtem Druck in einer Wirbelschicht gehalten werden. Entsprechend kann die Gesamtmenge oder eine Teilmenge aus Ausgangsstoff II getrennt von dem Wirbelschichtgas in den Wirbelschichtreaktor eingeleitet werden. Der Ausgangsstoff II wird zweckmäßig in einem beheizten Vorratsgefäß flüssig

gehalten und in einen Verdampfer dosiert, der dem Wirbelschichtreaktor vorgeschaltet ist. Gleichzeitig leitet man vorteilhaft einen schwachen Inertgasstrom, zweckmäßig von 5 000 bis 50 000 Volumenteilen Stickstoff oder des vorgenannten Kreisgases je Stunde, durch den Verdampfer. Der verdampfte Ausgangsstoff wird zusammen mit dem Kreisgasstrom durch das Katalysatorbett geleitet. Ebenfalls kann man aber auch den Ausgangsstoff II in fester Form, vorteilhaft in einer Korngröße zwischen 0,1 bis 3 Millimeter, oder in flüssiger Form direkt in den Wirbelschichtreaktor eindosieren, wobei die Zudosierung des Ausgangsstoffs II getrennt von dem Inertgas oder zusammen mit ihm erfolgen kann. Man kann das Verfahren nach der Erfindung in einem einfachen oder unterteilten, offenen oder geschlossenen Wirbelschichtsystem mit und ohne Fließstaubzirkulation durchführen. Bezüglich Reaktoren, Durchführung, Verfahrensvarianten und Reaktionsbedingungen des Wirbelschichtverfahrens wird auf Ullmanns Encyklopädie der technischen Chemie, Band 1, Seiten 916 bis 933, verwiesen. Die Aufarbeitung des Reaktionsgemisches erfolgt in vorgenannter Weise.

In einer vorteilhaften Ausführungsform führt man nach der Reaktion das dampfförmige Reaktionsgemisch in eine Trennkolonne, zieht aus dem Sumpf den Endstoff ab und führt über den Kopf der Kolonne das restliche Gemisch in eine Vorlage, wo die kondensierbaren Anteile, im wesentlichen Wasser und das dem Ausgangsstoff I zugrundeliegende Diamin kondensiert werden. Ein Teil des Abgases, zweckmäßig ein Anteil, der die vorgenannten Mengen an CO und $H_2$ enthält, wird in eine Waschkolonne geführt, anschließend getrocknet und als Kreisgas zurückgeführt.

In einer bevorzugten Ausführungsform wird im Wirbelbett umgesetzt und drucklos oder unter Druck, diskontinuierlich oder kontinuierlich gleichzeitig dem Wirbelreaktor ein Katalysatoranteil, zweckmäßig von 0,33 bis 10, vorzugsweise 2 bis 5 Gewichtsprozent Katalysator je Stunde Betriebsdauer des Wirbelbetts, bezogen auf die Gesamtmenge des Katalysators im Wirbelbett, entzogen und eine entsprechende, regenerierte Katalysatormenge zugeführt. Die Betriebsdauer des Wirbelbetts kann die Dauer eines kontinuierlichen Betriebs oder die Dauer des Betriebs mehrerer diskontinuierlicher Umsetzungen mit demselben Katalysator bedeuten. In einem kontinuierlichen Betrieb, insbesondere im Wirbelreaktor, wird in der Regel ein Verhältnis von 0,1 bis 3, vorzugsweise von 0,2 bis 1,7 Mol Nickeloxid und/oder Kobaltoxid, 0,1 bis 1,5, vorzugsweise 0,2 bis 0,6 Mol Molybdänoxid, 1 bis 30, vorzugsweise 1,6 bis 7,2 Mol Komponente c) je Stunde und Mol Ausgangsstoff II verwendet. Zuführung und Entnahme des Katalysators kann mit den üblichen Apparaturen des Feststofftransports, z. B. Förderpumpen, Transportschnecken erfolgen.

Der so entnommene Katalysator kann kontinuierlich, zweckmäßig im Kreislauf von der Entnahme über die Regenerierung zur Zuführung in das Wirbelbett, oder diskontinuierlich regeneriert werden. Die Regenerierung (Reaktivierung) des Katalysators erfolgt zweckmäßig ebenfalls in einem Wirbelreaktor. Die Schichthöhe im Regenerierungswirbelreaktor beträgt zweckmäßig 30 bis 2 000 Millimeter, die Verweilzeit des Katalysators im Regenerierungswirbelreaktor 10 bis 300, vorzugsweise 3 bis 50 Stunden. Als Wirbelschichtgas im Regenerierungswirbelreaktor (Regeneriergas) können die vorgenannten Inertgase, Gasgemische und kreisgas, gegebenenfalls unter Zuführung von Wasserdampf und Luft bzw. Sauerstoff verwendet werden. Die Regenerierung wird in der Regel bei einer Temperatur von 300 bis 600 °C, vorzugsweise 400 bis 500 °C, drucklos oder unter Druck, zweckmäßig mit 20 000 bis 500, vorzugsweise 2 000 bis 600 Gramm Inertgas bzw. Gasgemisch je Stunde und Kilogramm Katalysator mit Luft oder Sauerstoff, vorzugsweise in Mengen von 100 bis 10, insbesondere 70 bis 20 Mol Sauerstoff je Mol Komponente a) durchgeführt. Gegebenenfalls kann der Katalysator nach der Entnahme durch z. B. einen Zyklon, Elektrofilter als Kerzenfilter vom Katalysatorstaub befreit werden bzw. zusätzlicher Katalysator zum Ersatz vom Katalysatorverlusten der erfindungsgemäßen Reaktion wieder zugeführt werden. Vorteilhaft erlaubt diese Ausführungsform eine kontinuierliche Arbeitsweise ohne Betriebsunterbrechung wegen Regenerierung des Katalysators. Gebildeter Katalysatorstaub kann auf einfache Weise und ohne Verminderung der Katalysatoraktivität oder der Ausbeute durch die Regenerierung abgetrennt werden.

In einer bevorzugten Ausführungsform führt man das Verfahren in zwei Schritten durch und verwendet die N,N'-Diformyl-1,2-diamine als Ausgangsstoffe III. Bevorzugte Ausgangsstoffe III sind solche, in deren Formel $R^1$, $R^2$ und $R^4$ die bevorzugten Bedeutungen der Reste $R^1$, $R^2$ und $R^4$ der Ausgangsstoffe II besitzen. Die Umsetzung kann für den Fall der Verwendung von N,N'-Diformyl-1,2-diaminopropan durch die folgenden Formeln wiedergegeben werden :

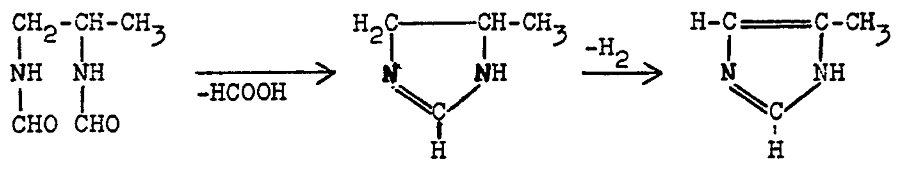


Im Vergleich zu den bekannten Verfahren liefert diese Verfahrensweise nach der Erfindung auf einfacherem und wirtschaftlicherem Wege 2-Imidazole, ausgehend von leicht zugänglichen Diaminen, in guter Ausbeute und Reinheit. Vorteilhaft ist es, daß das Reaktionsgemisch, das im ersten Schritt gebildetes Imidazolin enthält, die Aktivität des Katalysators (Komponenten a), b) und c)) des zweiten Schrittes nicht beeinträchtigt.

Als Ausgangsstoffe III kommen z. B. in Frage : die N,N'-Di-formylverbindungen von Äthylendiamin, 1, 2-Propylendiamin, 1,2-Butylendiamin, 1,2-Pentylendiamin, 1,2-n-Hexylendiamin, 1,2-n-Heptylendiamin, 1, 2-n-Octylendiamin, 1,2-n-Nonylendiamin, 1,2-n-Decylendiamin, 1,2-n-Octadecylendiamin ; 2,3-Butylendiamin, 2,3-Pentylendiamin, 2,3-Hexylendiamin, 2,3-Heptylendiamin, 2,3-Octylendiamin, 2,3-Nonylendiamin, 2,3-Decylendiamin, 3,4-Hexylendiamin, 3,4-Heptylendiamin, 3,4-Octylendiamin, 3,4-Nonylendiamin, 3,4-Decylendiamin, 4,5-Octylendiamin, 4,5-Nonylendiamin, 4,5-Decylendiamin, 5,6-Decylendiamin ; durch die Benzyl- und/oder Phenylgruppe in 1-Stellung einfach oder in 1- und 2-Stellung gleichzeitig substituierte Äthylendiamine ; durch vorgenannte Alkylgruppen in 1-Stellung und durch die Benzyl- oder Phenylgruppe in 2-Stellung substituierte Äthylendiamine ; die N-Methyl-, N-Äthyl-, N-Propyl-, N-Isopropyl, N-Butyl-, N-Isobutyl-, N-sek-Butyl-, N-tert.-Butyl-, N-Benzyl-, N-Phenyl-Verbindungen vorgenannter N,N'-Diformyl-1,2-diamine.

Die Umsetzung wird bei 200-400 °C in der Regel bei einer Temperatur von 200 bis 390 °C, zweckmäßig von 210 bis 370 °C, drucklos oder unter Druck, in der Regel bei mindestens 1 bar, zweckmäßig bei 1 bis 5, vorzugsweise 1,2 bis 2,5 bar, kontinuierlich druchgeführt. In der Regel dient das Reaktionsgemisch auch als Lösungsmedium, gegebenenfalls können auch vorgenannte, unter den Reaktionsbedingungen inerte, organische, zweckmäßig mit Wasser kein Azeotrop bildende Lösungsmittel verwendet werden.

Als Katalysator werden Zinkoxid oder Aluminiumoxid allein oder ein Gemisch von Zinkoxid und Aluminiumoxid, zweckmäßig in einem Verhältnis von Zink zu Aluminium wie 1 bis 50, vorzugsweise 8 bis 10 Grammatom Zink je Grammatom Aluminium, und von 0,1 bis 1 vorzugsweise von 0,2 bis 0,4 Grammatom Zink je Mol Ausgangsstoff III verwendet. Der Katalysator kann trägerfrei sein oder auch auf einem Träger, vorteilhaft in einer Menge von 1 bis 18 Gewichtsprozent Katalysator, bezogen auf den Träger, aufgebracht sein. Gleichzeitig kann $Al_2O_3$ als Katalysatorkomponente und für das Zinkoxid als Träger dienen. Bezüglich der Herstellung der Trägerkatalysatoren und der Katalysatorteilchen wird auf die vorgenannten Bedingungen der 3-Komponenten-Katalysatoren (a + b + c) verwiesen. Auch für den ersten Schritt gelten vorzugsweise die vorgenannten Bedingungen und Arbeitsweisen des zweiten Schrittes, insbesondere bezüglich Wirbelschicht, Teilchengröße, Schichthöhe des Katalysatorbettes, Verweilzeiten, Inertgasmenge, Verwendung von Stickstoff oder Kreisgas, Zusammensetzung des Kreisgases.

Die Reaktion in zwei Schritten kann wie folgt durchgeführt werden : Der dampfförmige Ausgangsstoff III, im Gemisch mit Inertgas, wird bei der Reaktionstemperatur des ersten Schrittes über den Katalysator bzw. Katalysator auf einem Träger in einem ersten Röhren- oder Wirbelschichtreaktor geleitet. Eine Verweilzeit von 1 bis 40, insbesondere 3 bis 20 Sekunden im Reaktionsraum des ersten Schrittes ist vorteilhaft. Das den Reaktor verlassende Reaktionsgemisch wird dann in vorgenannter Weise im zweiten Schritt an dem 3-Komponentenkatalysator (a + b + c) umgesetzt und der Endstoff in vorgenannter Weise isoliert. Entsprechend der vorgenannten Bedingungen, z. B. bezüglich Inertgasstrom und Arbeitsweise in der Wirbelschicht des zweiten Schrittes, kann auch der erste Schritt der Wirbelschicht durchgeführt werden. Die Verweilzeit beim ersten Schritt beträgt zweckmäßig 0,01 bis 20 Sekunden in der Wirbelschicht bzw. 3 bis 20 Sekunden im Festbett. In vorgenannter Weise können auch beide Schritte im Festbett oder vorteilhaft der 1. Schritt in der Wirbelschicht und der 2. Schritt im Festbett durchgeführt werden.

Die nach dem Verfahren der Erfindung herstellbaren Imidazole I sind wertvolle Ausgangsstoffe für die Herstellung von Farbstoffen, Pflanzenschutzmitteln, Textilhilfsmitteln, Katalysatoren für Polyurethane und Epoxidharze, oberflächenaktiven Mitteln und Pharmazeutika, z. B. den entsprechenden Nitroimidazolen. Imidazole der Formel I werden auch als Katalysatoren für Polymerisationsreaktionen und Aldolkondensationen verwendet. Bezüglich der Verwendung wird auf die vorgenannten Veröffentlichungen und Ullmanns Encyklopädie der technischen Chemie, (3. Auflage), Band 8, Seite 499, und (4. Auflage), Band 13, Seiten 173 bis 175, verwiesen.

Die in den folgenden Beispielen aufgeführten Teile bedeuten Gewichtsteile. Die Gewichtsteile verhalten sich zu den Volumenteilen wie Kilogramm zu Liter.

## Beispiel 1

Aus einem beheizten Dosiergefäß werden stündlich 75 Teile geschmolzenes 1-Phenylimidazolin in einen auf 300 °C erhitzten, horizontalen Quarzverdampfer dosiert. Die Dämpfe werden zusammen mit stündlich 5 000 Volumenteilen $N_2$ durch einen auf 400 °C beheizten Wirbelschichtreaktor geleitet. Der Wirbelreaktor ist ein vertikal auf dem Verdampfer sitzendes, elektrisch beheiztes Quarzrohr, das nach unten mit einer eingeschmolzenen Quarzfritte abgeschlossen ist. Das Quarzrohr ist mit 260 Teilen eines Katalysators aus 3 Gewichtsprozent Nickeloxid, 15 Gewichtsprozent Molybdänoxid, 77 Gewichtsprozent $\gamma$-Aluminiumoxid, 5 Gewichtsprozent $P_2O_5$ (Korngröße 0,1 bis 0,3 mm) zur Hälfte gefüllt. Die Verweilzeit in der Katalysatorzone im Wirbelzustand beträgt 1,5 Sekunden. Die Höhe der Katalysatorzone beträgt im Wirbelzustand 80 mm. Die den Reaktor verlassenden Dämpfe werden kondensiert und fraktioniert destilliert. Man erhält stündlich 69 Teile (95,8 % der Theorie, bezogen auf umgesetzten Ausgangsstoff II) 1-Phenylimidazol vom Kp (2 mbar) 110 bis 112 °C neben 2 Teilen nicht umgesetztem 1-Phenylimidazolin vom Fp 45 °C. Der Umsatz beträgt 97,3 Prozent, bezogen auf eingesetzten Ausgangsstoff II. Die Ausbeute bleibt noch nach 2 000 Stunden Betrieb konstant.

Beispiel 2

100 Teile 1,2-Diphenylimidazolin und 50 000 Volumenteile Stickstoff werden stündlich durch den auf 380 °C erhitzten Wirbelschichtreaktor geleitet. Man erhält analog Beispiel 1 stündlich 60,3 Teile (67,6 % der Theorie, bezogen auf umgesetzten Ausgangsstoff II) 1,2-Diphenylimidazol vom Fp 90 °C ; der Umsatz beträgt 90 Prozent, bezogen auf eingesetzten Ausgangsstoff II. Die Ausbeute bleibt noch nach 2 000 Stunden Betrieb konstant.

Beispiel 3

100 Teile 1-Phenyl-2-p-tolyl-imidazolin und 50 000 Volumenteile Stickstoff werden stündlich durch den auf 350 °C erhitzten Wirbelschichtreaktor geleitet. Man erhält analog Beispiel 1 stündlich 70,7 Teile (78,2 % der Theorie, bezogen auf umgesetzten Ausgangsstoff II) 1-Phenyl-2-p-tolylimidazol vom Fp 119 °C. Der Umsatz beträgt 91,2 %, bezogen auf eingesetzten Ausgangsstoff II. Die Ausbeute bleibt noch nach 2 000 Stunden Betrieb konstant.

Beispiel 4

100 Teile 1-Phenyl-2-(3',4'-dimethylphenyl)-imidazolin und 50 000 Volumenteile Stickstoff werden stündlich durch den auf 400 °C erhitzten Wirbelschichtreaktor geleitet. Man erhält analog Beispiel 1 stündlich 62,2 Teile (70,2 % der Theorie, bezogen auf umgesetzten Ausgangsstoff II) 1-Phenyl-2-(3',4'-dimethylphenyl)-imidazol vom Fp 146 bis 147 °C (aus Äthylalkohol). Der Umsatz beträgt 89,3 Prozent, bezogen auf eingesetzten Ausgangsstoff II. Die Ausbeute bleibt noch nach 2 000 Stunden konstant.

Beispiel 5

100 Teile 1-Phenyl-2-cyclohexylimidazolin und 50 000 Vulumenteile Stickstoff werden stündlich analog Beispiel 1 durch den auf 350 °C erhitzten Wirbelschichtreaktor geleitet. Man fraktioniert den Reaktoraustrag über eine Kolonne und erhält beim $Kp_{0,1}$ 118 °C 74,4 Teile 1-Phenyl-2-cyclohexylimidazol, entsprechend einer Ausbeute von 84,1 % der Theorie, bezogen auf umgesetzten Ausgangsstoff II. Der Umsatz beträgt 89,3 Prozent, bezogen auf eingesetzten Ausgangsstoff II. Die Ausbeute bleibt noch nach 2 000 Stunden Betriebsdauer konstant.

Beispiele 6 bis 10

Analog Beispiel 1 werden die in der Tabelle 1 aufgeführten Imidazole I erhalten.

(Siehe Tabelle 1, Seite 9 f.)

Tabelle 1

| Beispiel | Teile | Ausgangsstoff II | Temperatur °C | Teile Katalysator | Zusammensetzung in Gew.% | Endstoff | Fp (°C) | Ausbeute % der Theorie |
|---|---|---|---|---|---|---|---|---|
| 6 | 100 | 4,5-Dihydroimidazolin | 350 | 250 | 0,5 $Na_2O$<br>3 NiO<br>5 $P_2O_5$<br>15 $MoO_3$<br>76,5 $\gamma Al_2O_3$ | Imidazol | 90 | 82,1 |
| 7 | 100 | 2-Methyl-4,5-dihydroimidazolin | 370 | 280 | | 2-Methylimidazol | 145 | 89.3 |
| 8 | 100 | 2-Phenyl-4,5-dihydroimidazolin | 340 | 350 | | 2-Phenylimidazol | 145 | 88,2 |
| 9 | 100 | 4-Methyl-4,5-dihydroimidazolin | 380 | 280 | | 4-Methylimidazol | 45 | 84,7 |
| 10 | 100 | 2-Ethyl-4-methyl-4,5-dihydroimidazolin | 340 | 300 | | 2-Äthyl-4-methylimidazol | 38 | 83,5 |

Beispiel 11

a) 180 Teile 1,2-Diaminopropan werden unter Rühren und Kühlung mit 320 Teilen Ameisensäuremethylester bei 25 bis 30 °C gemischt. Nach dem Abkühlen des Reaktionsgemisches auf 0 °C wird das ausgefallene N,N'-Diformyldiaminopropan (Fp 65 bis 67 °C) abgesaugt und getrocknet.

b) 90 Teile N,N'-Diformyldiaminopropan werden pro Stunde aus einem Vorratsgefäß zusammen mit 600 000 Teilen Stickstoff pro Stunde durch einen auf 360 °C erhitzten Wirbelreaktor geleitet. Der Wirbelreaktor ist ein vertikales, elektrisch beheiztes Quarzrohr, das nach unten mit einer Quarzfritte abgeschlossen ist. An den Wirbelreaktor ist ein Festbettreaktor mit externer Bezeizung als zweite Reaktionsstufe angeschlossen. Die Verweilzeiten betragen in der ersten Stufe 1,7 Sekunden, in der zweiten Stufe 6 Sekunden. Die Reaktionstemperaturen sind 330 °C (1. Stufe) und 360 °C (2. Stufe), die Höhe der Wirbelschicht beträgt 80 Millimeter, die des Katalysators im Festbett 600 Millimeter. Der Katalysator in der 1. Stufe enthält 300 Teile eines Katalysators aus 90 Gewichtsprozent Zinkoxid und 10 Gewichtsprozent Aluminiumoxid (Porenvolumen 0,5 Milliliter je Gramm ; Schüttgewicht 1,1 Gramm je Milliliter ; Korngröße 0,1 bis 0,3 Millimeter). Der Katalysator in der 2. Stufe enthält 910 Teile eines Katalysators aus 3,1 Gewichtsprozent Nickeloxid, 17,5 Gewichtsprozent Molybdäntrioxid und 5,8 Gewichtsprozent $P_2O_5$, 73,6 Gewichtsprozent γ-Aluminiumoxid ; Strangform von 1,5 Millimeter Durchmesser. Die den Reaktor verlassenden Dämpfe werden kondensiert und fraktioniert destilliert. Man erhält stündlich 46 Teile (81 % der Theorie, bezogen auf eingesetzten Ausgagsstoff III) 4-Methylimidazol vom Fp 45 °C. Der Umsatz beträgt 95,3 Prozent. Die Ausbeute bleibt auch noch nach 2 000 Stunden Betrieb konstant.

Beispiele 12 bis 15

Analog Beispiel 11 werden die folgenden, in der Tabelle 2 aufgeführten Umsetzungen durchgeführt.

Tabelle 2

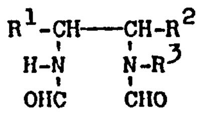

| Beispiel | Ausgangsstoff III Teile/Stunde | Teile $N_2$/Stunde | $R^1$ | $R^2$ | $R^3$ | Temperatur 1.Stufe (°C) | Teile Katalysator 1.Stufe | Zusammensetzung in Gew.% |
|---|---|---|---|---|---|---|---|---|
| 12 | 80 | 600 000 | H | H | H | 300 | 250 | |
| 13 | 90 | 600 000 | H | $CH_3$ | H | 250 | 250 | 10 % γ-Aluminiumoxid |
| 14 | 80 | 600 000 | H | H | H | 300 | 250 | 90 % Zinkoxid |
| 15 | 80 | 600 000 | H | H | H | 300 | 250 | |

Tabelle 2 (Fortsetzung)

| Beispiel | Temperatur 2. Stufe (°C) | Teile Katalysator 2. Stufe | Zusammensetzung (Gew.%) | Ausbeute an Endstoff I in % der Theorie | Fp in (°C) |
|---|---|---|---|---|---|
| 12 | 340 | 600 | 6 NiO | 80,2 | 90 |
| 13 | 360 | 1 000 | 15 $MoO_3$<br>79 $MgSiO_3$ | 78,3 | 45 |
| 14 | 335 | 620 | 5,1 CoO | 77,3 | 90 |
| 15 | 340 | 600 | 13,4 $MoO_3$<br>81,5 γ-Aluminiumoxid | 78,2 | 90 |

Beispiel 16

Stündlich werden 50 Teile N,N'-Diformyldiaminopropan in einem Quarzverdampfer bei 300 °C verdampft und die Dämpfe zusammen mit 310 000 Volumenteilen pro Stunde Kreisgas durch einen Wirbelreaktor und anschließend durch einen Festbettreaktor geleitet. Das Kreisgas enthält 73,4

Gewichtsprozent CO, 6,55 Gewichtsprozent $H_2$, 2,3 Gewichtsprozent $NH_3$, 3,3 Gewichtsprozent Methan und 14,45 Gewichtsprozent $CO_2$. Die 1. Stufe (Eingangsstufe, Wirbelschicht) ist mit 250 Teilen Katalysator, die 2. Stufe (Festbett) mit 700 Teilen Katalysator gefüllt. Die Verweilzeiten betragen in der 1. Stufe 2,6 Sekunden, in der 2. Stufe 8 Sekunden. Die Reaktionstemperaturen sind 330 °C (1. Stufe) und 350 °C (2. Stufe), die Höhe der 1. Wirbelschicht beträgt 80 Millimeter, die des Festbetts 1 000 Millimeter. Der Reaktor in der 1. Stufe enthält einen Katalysator (Korngröße 0,1 bis 0,3 Millimeter ; Porenvolumen 0,3 Milliliter je Gramm ; spezifische Oberfläche 180 Quadratmeter je Gramm ; Schüttgewicht 1 Gramm je Milliliter), der aus 80 Teilen ZnO und 20 Teilen γ-Aluminiumoxid besteht. Der Reaktor in der 2. Stufe (Festbettreaktor) enthält 700 Teile eines Katalysators in Strangform von 1,5 Millimeter Durchmesser, der aus 3,1 Gewichtsprozent Nickeloxid, 17,5 Gewichtsprozent Molybdäntrioxid und 5,8 Gewichtsprozent $P_2O_5$, 73,6 Gewichtsprozent γ-Aluminiumoxid besteht. Nach der Aufarbeitung analog Beispiel 1 erhält man stündlich 21,5 Teile (82,3 % der Theorie, bezogen auf eingesetzten Ausgangsstoff III) Imidazol vom Schmelzpunkt 90 °C. Der Umsatz beträgt 96,5 Prozent, bezogen auf das eingesetzte Diamid. Die Ausbeute bleibt auch noch nach 2 000 Stunden konstant. Ein Anteil der Abgase wird als vorgenanntes Kreisgas in den Wirbelreaktor zurückgeführt.

**Ansprüche**

1. Verfahren zur Herstellung von Imidazolen der Formel (I)

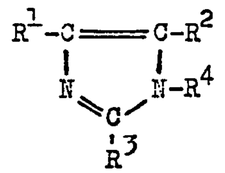

(I)

worin $R^1$, $R^2$, $R^3$ und $R^4$ gleich oder verschieden sein können und jeweils einen Alkylrest mit 1 bis 18 Kohlenstoffatomen, einen Allylrest, einen Crotylrest, einen Oleylrest, einen n-Undecen-(11)-yl-(1)-rest, einen Cycloalkylrest mit 5 bis 7 Kohlenstoffatomen, einen Aralkylrest oder Alkylarylrest mit 7 bis 12 Kohlenstoffatomen, einen Phenylrest oder ein Wasserstoffatom bedeuten, durch Umsetzung von Imidazolinen in Gegenwart von Metallkatalysatoren bei einer Temperatur von 250 bis 500 °C, dadurch gekennzeichnet, daß man 2-Imidazoline der Formel (II)

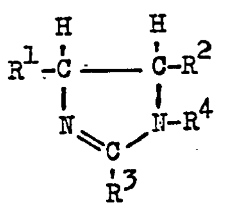

(II)

worin $R^1$, $R^2$, $R^3$ und $R^4$ die vorgenannte Bedeutung besitzen, in Gegenwart von Inertgas oder solche Inertgase enthaltendem Kreisgas und in Gegenwart eines Katalysators oder gegebenenfalls Trägerkatalysators, dessen aktive Komponente aus

a) Molybdänoxid,

b) Nickeloxid und/oder Kobaltoxid,

c) Aluminiumoxid, Aluminiumsilikat, Siliziumdioxid, Magnesiumaluminium silikat hydrat, Dimagnesiumaluminiumsilikathydrat, Natriumaluminiumsilikat, Calciumaluminiumsilikat und/oder Magnesiumsilikat im Molverhältnis a : b : c wie 1 : (0,5-5) : (5-50) und

d) gegebenenfalls Sauerstoffverbindungen des Zinks, Magnesiums, Chroms, Phosphors und/oder Natriumoxid besteht, umsetzt, wobei die Katalysatormenge pro Mol Ausgangs-2-Imidazolin der Formel (II) 0,1-1,5 Mol Molybdänoxid entspricht.

2. Verfahren zur Herstellung von Imidazolen, wobei man in einem ersten Schritt N,N'-Diformyl-1,2-diamine der Formel (III)

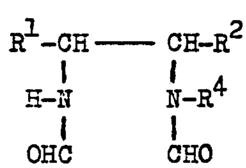

(III)

worin $R^1$, $R^2$ und $R^4$ die vorgenannte Bedeutung besitzen, bei einer Temperatur von 200 bis 400 °C in Gegenwart von Zinkoxid und/oder Aluminiumoxid als Katalysatoren in der Gasphase umsetzt, dadurch gekennzeichnet, daß man nach dem ersten Schritt dann das so erhaltene, 2-Imidazolin (II) enthaltende

Reaktionsgemisch in einem zweiten Schritt nach dem Verfahren nach Anspruch 1 umsetzt.

3. Verfahren nach Anspruch 1, dadurch gekennzeichnet, daß die Umsetzung in Gegenwart von Katalysatoren mit einer Teilchengröße von 0,05 bis 7 Millimeter durchgeführt wird.

4. Verfahren nach Anspruch 1, dadurch gekennzeichnet, daß die Umsetzung im Festbett mit Verweilzeiten der Ausgangsstoffe (II) in der Katalysatorschicht von 0,2 bis 20 Sekunden und in der Wirbelschicht mit Verweilzeiten von 0,01 bis 20 Sekunden durchgeführt wird.

**Claims**

1. A process for the preparation of imidazoles of the formula (I)

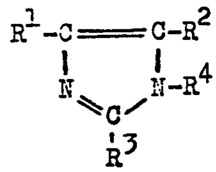

(I)

where $R^1$, $R^2$, $R^3$ and $R^4$ may be identical or different and each is alkyl of 1 to 18 carbon atoms, allyl, crotyl, oleyl, n-undec-11-en-1-yl, cycloalkyl of 5 to 7 carbon atoms, aralkyl of alkylaryl of 7 to 12 carbon atoms, phenyl or hydrogen, by reacting imidazolines in the presence of a metal catalyst at a temperature of from 250 to 500 °C, wherein a 2-imidazoline of the formula (II)

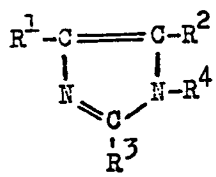

(II)

where $R^1$, $R^2$, $R^3$ and $R^4$ have the above meanings, is reacted in the presence of inert gas or a circulating gas containing inert gas and in the presence of an unsupported catalyst or a supported catalyst whose active component consists of

a) molybdenum oxide,

b) nickel oxide and/or cobalt oxide, and

c) aluminum oxide, aluminum silicate, silicon dioxide, magnesium aluminum silicate hydrate, dimagnesium aluminum silicate hydrate, sodium aluminum silicate, calcium aluminum silicate and/or magnesium silicate, the molar ratio of a) to b) to c) being 1 : (0.5-5) : (5-50), with or without

d) oxygen compounds of zinc, magnesium chromium, phosphorus and/or sodium oxide, the amount of catalyst being such that the molar ratio of molybdenum oxide to starting material 2-imidazoline of the formula II is 0.1-5:1.

2. A process for the preparation of imidazoles, in which, in a first step, an N,N'-diformyl-1,2-diamine of the formula (III)

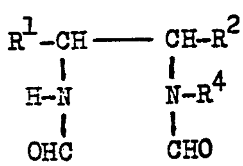

(III)

where $R^1$, $R^2$ and $R^4$ have the above meanings, is reacted in the gas phase, at a temperature of from 200 to 400 °C, in the presence of zinc oxide and/or aluminum oxide as the catalyst, wherein, after the first step, the resulting reaction mixture, containing a 2-imidazoline (II) is then reacted, in a second step, in accordance with the process as claimed in claim 1.

3. A process as claimed in claim 1, wherein the reaction is carried out in the presence of a catalyst having a particle size of from 0.05 to 7 millimeters.

4. A process as claimed in claim 1, wherein the residence time of the starting material (II) in the catalyst bed is from 0.2 to 20 seconds in the case of the reaction carried out in a fixed bed, and from 0.01 to 20 seconds in the case of the reaction carried out in a fluidized bed.

**Revendications**

1. Procédé pour la préparation d'imidazoles de formule (I)

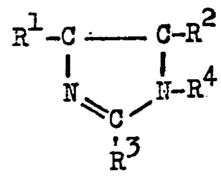

(I)

dans laquelle $R^1$, $R^2$, $R^3$ et $R^4$ peuvent être semblables ou différents et représentent un radical alcoyle à 1-18 atomes de carbone, un radical allyle, un radical crotyle, un radical oléyle, un radical n-undécène-(11)-yle-(1), un radical cycloalcoyle à 5-7 atomes de carbone, un radical aralcoyle ou alcoylaryle à 7-12 atomes de carbone, un radical phényle ou un atome d'hydrogène, par réaction d'imidazolines en présence de catalyseurs métalliques à une température de 250 à 500 °C, caractérisé en ce qu'on fait réagir des 2-imidazolines de formule (II)

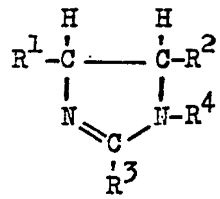

(II)

dans laquelle $R^1$, $R^2$, $R^3$ et $R^4$ ont les significations données ci-dessus, en présence de gaz inerte ou de gaz de recyclage qui contiennent un tel gaz inerte et en présence d'un catalyseur ou, le cas échéant, d'un catalyseur fixé sur support, dont le constituant actif se compose de :

a) oxyde de molybdène,

b) oxyde de nickel et/ou oxyde de cobalt,

c) oxyde d'aluminium, silicate d'aluminium, bioxyde de silicium, silicate d'aluminium-magnésium hydraté, silicate d'aluminium dimagnésien hydraté, silicate d'aluminium-sodium, silicate d'aluminium-calcium et/ou silicate de magnésium, dans le rapport molaire a : b : c = 1 : (0,5-5) : (5-50) et

d) le cas échéant, composés oxygénés du zinc, du magnésium, du chrome, du phosphore et/ou oxyde de sodium, la quantité de catalyseur par mol de 2-imidazoline de départ de formule (II) correspondant à 0,1-1,5 mol d'oxyde de molybdène.

2. Procédé pour la préparation d'imidazoles, dans lequel on fait réagir en phase gazeuse, dans un premier temps, des N,N'-diformyl-1,2-diamines de formule (III)

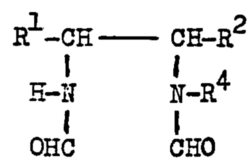

(III)

dans laquelle $R^1$, $R^2$ et $R^4$ ont les significations données ci-dessus, à une température de 200 à 400 °C en présence d'oxyde de zinc et/ou d'oxyde d'aluminium servant de catalyseurs, caractérisé en ce qu'à la suite du premier temps, on fait réagir le mélange réactionnel ainsi obtenu, contenant de la 2-imidazoline (II), en un second temps suivant le procédé de la revendication 1.

3. Procédé selon la revendication 1, caractérisé en ce que la réaction est menée en présence de catalyseurs ayant une grosseur de particules de 0,05 à 7 mm.

4. Procédé selon la revendication 1, caractérisé en ce que la réaction est menée avec des durées de séjour des substances de départ (II) dans la couche de catalyseur de 0,2 à 20 secondes en lit fixe et de 0,01 à 20 secondes en couche fluidisée.